# EUROPEAN PATENT APPLICATION

(11) **EP 3 756 678 A1**
(43) Date of publication of application: **30.12.2020**
(21) Application number: 19189682.8
(22) Date of filing: 01.08.2019
(51) Int. Cl.: A61K 36/28

(54) **METHOD FOR OBTAINING CYNAROPICRIN-RICH EXTRACTS**

(30) Priority: 28.06.2019 PT 2019115609
(71) Applicant: Cebal - Centro De Biotecnologia Agrícola E Agro-Alimentar Do Alentejo, 7801-908 Beja (PT)
(72) Inventor: AMOREIRA SOUSA SILVA BRÁS, TERESA ISABEL, 7600-017 ALJUSTREL (PT); PEREIRA DUARTE RICARDO, MARIA DE FÁTIMA, 7800-073 BEJA (PT); GRAÇA NEVES, LUÍSA ALEXANDRA, 2820-142 CHARNECA DA CAPARICA (PT); SEREJO GOULÃO CRESPO, JOÃO PAULO, 1300-319 LISBOA (PT); COSTA PAULINO, ANA FILIPA, 7800-258 BEJA (PT); FIRMINO ROSA, DANIELA FILIPA, 7800-326 BEJA (PT)
(74) Representative: Ferreira, Maria Silvina

(57) **Abstract**

This application relates to a method for obtaining cynaropicrin-enriched extracts, from leaves of *Cynara cardunculus* (cardoon).

In order to obtain a cynaropicrin-rich extract, the present patent application discloses steps cynaropicrin and carbohydrates, taking into account the similar molecular weight of compounds. The method disclosed herein comprises steps of grounding air dried leaves of *Cynara cardunculus,* pulsed ultrasonic assisted extraction and diananofiltration using membranes specific for cynaropicrin and carbohydrate separation.

The presently described method allows the production of low sugars cardoon extract rich in cynaropicrin to be used in nutraceuticals.

## Description

### Technical field

This application relates to a method for obtaining cynaropicrin-enriched extracts.

### Background art

In a concept of natural resources economical and biological valorization, where plants represent a wide group, chemical analysis of their whole morphological parts could indicate the best way for its valorization. With flowers typically applied as rennet for cheese manufacturing and with a biomass production that can go from 7.8 till 20 ton DW/ha, *Cynara cardunculus* (cardoon) research interest has grown in a perspective of plant total exploitation, presenting potential as source of lignocellulosic biomass (stems and whole plant), as a biomass for oil production (seeds), and as a source of bioactive compounds (leaves and roots), with the last one representing from 33.1 till 48.4% regarding the total biomass average weight [1-3].

Presenting a high biological potential, with antiinflammatory, anti-proliferative or anti-photoaging activities as examples, sesquiterpene lactones can be found in lipophilic cardoon leaves extracts in amounts that can go up to 95 mg/g DW with cynaropicrin as the most abundant sesquiterpene lactone found (87 mg/gDW).

The patent document EP3466936 discloses that, upon cynaropicrin from cardoon leaves extraction optimization revealed that pulsed ultrasound assisted extraction (PUAE) and ethanol with extraction solvent, allowed the best extraction yield and highest cynaropicrin concentration and obtained extracts chemical characterization revealed a high content of carbohydrates, that could achieve 30% of extract weight [4, 5]. Concerning the achievement of an extract rich on cynaropicrin and compounds with high biological activity, carbohydrates removal can promote an increase on economic and biological crude extracts value as well as the production of an extract rich in carbohydrates.

Plant extracts fractionation and/or compounds purification, namely cynaropicrin, is typically made towards the application of solvents with different selectivity and affinities to target compounds and using chromatographic systems and traditional systems for compounds concentration, as distillation, generally makes use of high temperatures, what is unappropriated for heat-sensitive products, besides that also present high energy consumptions [6-9].

In the last years, nanofiltration has been successfully applied for fractionation and concentration of different compounds and for the best of our knowledge there is disclosed data regarding the removal of sugars and concentration of cardoon PUAE ethanolic extracts.

### Summary

This application relates to a method for obtaining cynaropicrin-enriched extracts, from leaves of *Cynara cardunculus,* wherein said method comprises the following steps:
- Grounding of air dried *Cynara cadunculus;*
- Pulsed Ultrasonic assisted extraction (PUAE);
- Diananofiltration of the extract obtained in the previous step at a transmembrane pressure between 12 and 40 bar and a crossflow velocity between 0.5 and 1.5 m/s, wherein the membranes used for the filtration have a molecular weight cut-off between 100 and 400 Da;
wherein the retentate is an extract rich in cynaropicrin.

In one embodiment, the method for obtaining a cynaropicrin-rich extract described above further comprises a post-concentration step; wherein the permeate is removed until volume reduction factor (VRF) of 2 is achieved.

In one embodiment, the Ultrasonic assisted extraction step is performed with a duty cycle of 25%, a Solid/Liquid ratio of 1/27, extraction temperature of 44 °C and amplitude of 67% during 30 minutes.

In one embodiment, the membrane used in the diananofiltration step is made of modified polyimide or polyamide thin-film composite.

In one embodiment, the diananofiltration step is performed at a temperature between 25 and 40 °C.

### Detailed Description

The present application provides a method for obtaining cynaropicrin-rich extracts by diananofiltration.

In order to obtain a cynaropicrin-rich extract, the present patent application discloses steps for sugars (mainly glucose and fructose) and cynaropicrin separation, taking into account the similar molecular weight of compounds (180 g mol⁻¹ for glucose and 346 g mol⁻¹ for cynaropicrin) and the available membranes as well as the solvent (ethanol) effect on membranes selected, that could affect their compounds selectivity.

Diananofiltration consists in continuously feed fresh solvent to feed solution at the same rate as the permeate is recovered, allowing a total depletion of low molecular weight compounds [10]. However, there is little information regarding Diananofiltration applied to solvent resistant nanofiltration (SRNF) in a total organic solvent environment. Thus, it is also disclosed herein the application of SRNF in diananofiltration mode for cardoon PUAE extracts sugars (glucose and fructose) removal, with a cynaropicrin maximum loss of 10%, followed by extract concentration until a maximum volume reduction factor of 2. This methodology was defined with the objective of producing one low sugars cardoon extract rich in cynaropicrin for nutraceuticals market.

The method for obtaining a cynaropicrin-rich extract disclosed herein comprises the following steps:
- Grounding of air dried *Cynara cadunculus;*
- Ultrasonic assisted extraction (UAE);
- Diananofiltration of the extract obtained in the previous step at a transmembrane pressure between 12 and 40 bar and a crossflow velocity between 0.5 and 1.5 m/s, wherein the membranes used for the filtration have a molecular weight cut-off between 100 and 400 Da;
wherein the retentate is an extract rich in cynaropicrin.

In one embodiment, the method for obtaining a cynaropicrin-rich extract described above further comprises a post-concentration step; wherein the permeate is removed until volume reduction factor (VRF) of 2 is achieved.

By "extract rich in cynaropicrin" or "cynaropicrin-rich extract" it should be understood as an extract with a cynaropicrin concentration of at least 7% (w/w) and a maximum sugars concentration of 2% (w/w).

### Materials and Methods

### 1.1. Reagents and standards

Extraction solvents used were Ethanol absolute, both provided by BDh Prolabo (France). Acetonitrile (HPLC grade) and H2SO₄ were both provided by Merck (France). Standard of cynaropicrin was acquired at Extrasynthese (France) and glucose (≥99,5%) and fructose (≥99%) were obtained from SIGMA (Germany).

### 1.2. Raw material

*Cynara cardunculus* L. (DC) leaves were collected in June 2015 at the Experimental Center of Agriculture School of Instituto Politecnico de Beja, Portugal and preserved at - 80 °C. Before extraction, samples were air dried at room temperature till constant weight. Dried leaves were grounded using a domestic mixed grinder (Moulinex).

### 1.3. Extraction

Pulsed ultrasonic assisted extraction (PUAE) experiments were performed using an UAE probe device (Bandelin HD3200, VS70T probe, Germany), 20 kHz. Extraction was made as described by Bras et al. [5] . Briefly, pulsed ultrasound assisted extraction, with a duty cycle of 25%, a S/L ratio of 1/27, extraction temperature of 44 °C and amplitude of 67% was performed during 30 minutes. After extraction, the extract was filtered in a glass filter funnel, with porosity G4 and 10-16 µm maximum nominal pore size. Ethanol was evaporated in a rotary evaporator (Hei-VAP Advantage, Heidolph, Germany).

### 1.4. Nanofiltration experiment

### 1.4.1. Experimental setup

The nanofiltration experimental setup used in the present patent application is shown in Figure 1. It is comprised by a GE-Sepa CF cross-flow module (GE Osmonics, USA) and a highpressure feed pump (Hydra-cell model G13, Wanner Engineering Inc., USA). The effective membrane area used was 140 cm².

In the context of the present patent application and considering target compounds molecular weight, NF90 (DOW, USA) and NF270 (DOW, USA) membranes were tested. Additionally, an organic solvent nanofiltration (OSN) membrane, Duramem 200 (Evonik, Germany) was also used. Membranes properties are listed in table 1.

**Table 1. Characteristics of NF90, NF270 and Duramem 200 membranes**

| | **NF90** | **NF270** | **Duramem 200** |
|---|---|---|---|
| Manufacturer | Dow/Filmtec | Dow/Filmtec | Evonik |
| Surface material | Polyamide | Polyamide | Modified Polyamide |
| Molecular Weight cut-off (Da) | 100 [14] | 400 [14] | 200 [15] |
| Maximum temperature (°C) | 45 | 45 | 50 |
| Maximum pressure (bar) | 41 | 41 | 60 |

### 1.4.2. Membrane selection

Considering the membrane selection for the best cynaropicrin recovery, membranes under study were firstly characterized in terms of: ethanol and ethanolic extract permeability; cynaropicrin, glucose and fructose rejections and massic swelling degree. During permeability and rejection experiments, system was operated under a total recirculation mode, at a transmembrane pressure range between 4 and 20 bar and a temperature of 30 °C, controlled by an external refrigerator bath (Model 89203-012, VWR International, USA). Samples of permeate and feed were collected for each pressure value and cynaropicrin, glucose and fructose concentrations were quantified, and apparent rejection values were determined.

Permeability and rejections were calculated using the following equations, respectively: ${J}_{v} = {L}_{p} \left(ΔP-Δπ\right)$ (equation 1) ${R}_{i} = 1 - \frac{{C}_{i , p}}{{C}_{i , f}}$ (equation 2) Where Jᵥ is the solvent volumetric flux (m³.m⁻².h⁻¹), Lp, is the membrane permeability (m³.m⁻².h⁻¹.bar), ΔP is the transmembrane pressure (bar), and Δπ is the osmotic pressure difference (bar), which is determined by the Van't Hoff equation. Rᵢ is the apparent rejection of solute i (%), and *C_{i,p}* (g.m-3) and *C_{i,f}* (g.m⁻³) are the concentration in the permeate and feed of solute i, respectively.

Pre-weighed pieces (10 x 10 mm) of dry membrane were immersed in ethanol and allowed to stand at 30 °C during 24 h, where after this, they were removed, the liquid excess wiped and weighted again. The swelling degree of the membrane was calculated by: ${S}_{D} = \frac{{W}_{S} - {W}_{D}}{{W}_{D}} \times 100$ (equation 3) Where S_{D} is the membrane swelling degree (%), W_{D} (g) is the weight of the dried membrane and W_{S} (g) is the weight of the swollen membrane.

### 1.4.3. Diananofiltration

During the diananofiltration experiment, a feed volume of 1.475 L, a recirculation flow of 240 L and a transmembrane pressure of 20 bar were used. Feed volume V_{feed} (L) was kept constant by the addition of a volume of ethanol, V_{EtOH} (L) and the number of Diavolumes, D (-), were calculated as the ratio between the washing solvent volume V_{EtOH} and the initial feed volume, V_{feed}. Feed, cumulative and instantaneous permeate samples were collected over time, cynaropicrin, glucose and fructose concentrations were quantified, and compounds apparent rejections were determined (equation 2). When operating in a diafiltration mode, concentration of compound i in feed vessel, can be obtained by a mass balance to the system: $\frac{d \left({V}_{f}{C}_{i , f}\right)}{dt} = - {J}_{v} {AC}_{i , p}$ (equation 4)

Thus ${C}_{if} \frac{dVt}{dt} + {V}_{f} \frac{{dC}_{i , f}}{dt} = - {J}_{v} {AC}_{i , p}$ (equation 5)

Since the volume is constant, Eq. 4 can be written as ${\int }_{t = 0}^{t = t} dt = - \frac{{V}_{f}}{{AJ}_{v}} {\int }_{{C}_{i , f , t = 0}}^{{C}_{if , t = t}} \frac{1}{{C}_{if} \left(1-R\right)} {dC}_{i , f}$ (equation 6)

In this case, to simplify, Rejection (R) was considered constant during the experiment. So the Eq. 5, can be solved as: ${C}_{i , f} = {C}_{i , {f}_{o}} exp \left(-\frac{{J}_{v} A}{{V}_{f} \left(1-R\right)}t\right)$ (equation 7) Where, V_{f} is the feed volume (m³), C_{i,f} is the concentration in feed of solute i (mol.m⁻³), Ci,p is the concentration in the feed of solute i (mol.m⁻³), Jᵥ is the permeate volumetric flux (m³.m⁻².h⁻¹), A is the membrane area (m²) and t (h) is the diafiltration time.

### 1.4.4. Post - concentration

In order to decrease feed volume and increase cynaropicrin concentration, a post-concentration step was made, where permeate was continuously removed until a volume reduction factor, VRF (-), of 2 was achieved. VRF during the concentration experiment was calculated using the following equation: $VRF = \frac{{V}_{r}}{{V}_{f}}$ (equation 8) Where, VRF corresponds to the volume reduction factor (m³), Vᵣ is the retentate volume (m³) and V_{f} is the feed volume in the beginning of the experiment (m³).

### 1.5. Cynaropicrin quantification by High Pressure Liquid Chromatography (HPLC)

Cynaropicrin was quantified by HPLC. A Dionex Ultimate 3000 system (Thermo Scientific, USA), equipped with a Diode Array detector DAD-3000 (Thermo Scientific, USA) was used. A Kinetex F5 2.6µ (4.6 x 150 mm) column, from Phenomenex (USA), was used at 30 °C, with water:acetonitrile (75:25) as mobile phase, at a flow rate of 0.5 mL/min. All samples were pre-filtered with 0.22 µm pore size membrane filters (Pall, USA) .

### 1.6. Monosaccharides quantification by High Pressure Liquid Chromatography (HPLC)

Glucose and fructose were quantified by HPLC. A Dionex Ultimate 3000 system (Thermo Scientific, USA), equipped with a Refraction Index ERC RefractoMax 520 (Thermo Scientific, USA) was used. An Aminex HPX-87H (7.8 x 300 mm) cation exchange column, from Bio-Rad (7.8 x 300 mm) was used at 50 °C, using 5 mM H₂SO₄ solution as mobile phase at a flow rate of 0.6 mL/min. All samples were pre-filtered with 0.22 µm pore size membrane filters from Pall, USA).

### 3. Results and discussion

### 3.1. Membrane selection

Extract permeability values indicated that Duramem200 presents the lowest extract permeability, followed by NF90 and NF270 membranes. Although the NF270 membrane presented the highest extract permeability, cynaropicrin (32%) and sugars (25%) apparent rejections were very low comparatively to the other membranes under study, mainly due to its molecular weight cut-off (MWCO) of 400 Da (table 1) and to the highest swelling degree (51.63%), turning this membrane not suitable for cynaropicrin and sugars separation.

**Table 2. Values for ethanol and extract permeability, cynaropicrin, glucose and fructose rejections at 20 bar and massic swelling for NF90, NF270 and Duramem 200 membranes.**

| **Membrane** | **Lp_{EtOH} (L/m². h.bar)** | **Lp_{Extract} (L/m². h.bar)** | **Rᵢ (%) (20 bar)** | | | **Massic swelling** |
|---|---|---|---|---|---|---|
| | | | Cynaropicrin | Glucose | Fructose | |
| **NF90** | 3.15 | 0.65 | 93 | 86 | 83 | 41.35% |
| **NF270** | 2.30 | 0.76 | 32 | 26 | 24 | 51.63% |
| **Duramem 200** | 0.76 | 0.25 | 98 | 81 | 76 | 17.12% |

Considering the extract permeabilities and compounds apparent rejections, a mass balance using equation 7, could be used for membrane behavior prediction in diananofiltration mode. The mass balance was applied considering a feed volume V_{f} of 1.5 L and a cynaropicrin maximum loss of 10% comparatively to the cynaropicrin initial concentration and results are represented in table 3. An efficient process separation is the one that promotes the highest removal of non interest compound and the lowest loss of target compound. Although cynaropicrin apparent rejection for NF90 and Duramem 200 were very similar, sugars apparent rejection was lower for Duramem 200 and for a maximum cynaropicrin loss of 10%, a removal of 75% and 78% of glucose and fructose respectively, is predicted to be obtained with Duramem 200 contrarily to 22% glucose and fructose loss when using NF90. Considering the wash solvent volume, expressed in terms of diavolumes (V_{D}), for the same target of 10% cynaropicrin maximum loss, 5 V_{D} would be needed considering the Duramem 200 membrane, against 1.82 V_{D} with NF90 membrane. Considering the 75% of glucose removal obtained with Duramem200, a cynaropicrin loss of 50% would be achieved with NF90 membrane with a consumption of 9.83 V_{D}.

**Table 3. Predicted massic balance for compounds under study (cynaropicrin, glucose and fructose) for NF90 and Duramem 200 membranes with a weight loss of 10% cynaropicrin and with 75% glucose weight loss for NF90 membrane.**

| **Membrane** | **W_{loss, cyn} (%)** | **W_{loss, glucose} (%)** | **W_{loss, fructose} (%)** | **V_{D}** |
|---|---|---|---|---|
| **NF90** | 10 | 22 | 22 | 1.82 |
| **Duramem 200** | 10 | 75 | 78 | 5 |
| **NF90** | 50 | 75 | 75 | 9.83 |

Considering, a better separation obtained with the Duramem 200, this membrane was the one selected for diananofiltration experiments with *Cynara cardunculus* ethanolic extract.

### 3.2. Diananofiltration experiment

### 3.2.1. Permeate flux during diananofiltration

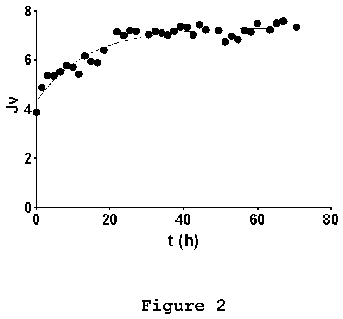

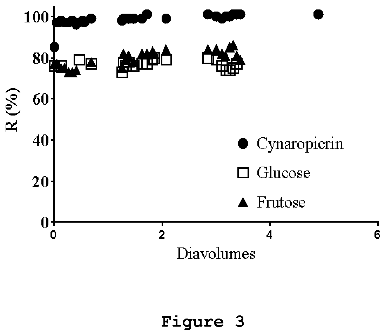

The *Cynara cardunculus* ethanolic extract was processed in a diananofiltration mode at 20 bar and 35 °C. Figure 2 shows the variation of permeate flux with diavolumes and its possible to observe that after approximately 24 h an increase of 50% on permeate flux. In order to understand the influence of the permeate flux increase, apparent rejections for compounds under study during diananofiltration were analyzed, with no significant differences on compounds rejection (figure 3), indicating that the increase on permeate flux did not influence compounds rejection and thus, mass balance assumptions by equation 7 are still valid. For a correct diananofiltration simulation, permeate flux, Jᵥ, was calculated considering the data obtained with figure 2 and the mathematical fit to the experimental data gives that: ${J}_{V} = 7.333 - 3.048 {e}^{- 0.075 t}$ (equation 9) with an experimental fit error of 0.9053.

Diananofiltration experiment was made considering a maximum removal of sugars with an enrichment of *Cynara cardunculus* ethanolic extract in cynaropicrin (1.62 g/L) with a maximum loss of 10% weight. Comparison between experimental data and the predicted one according to equation 7 and assuming compounds rejection constant as mentioned above, shows that experimental cynaropicrin 9.27% weight loss was achieved at 5.73 diavolumes with a removal of 56.63% and 64.89% of glucose and fructose (table 4), respectively, with experimental data fitting the predicted one.

**Table 4. Experimental massic balance for cynaropicrin, glucose and fructose for diananofiltration experiment.**

| | **Weight (mg)** | | |
|---|---|---|---|
| | **Initial** | **Final** | % **weight loss** |
| **Cynaropicrin** | 2194 | 1990 | 9,27% |
| **Glucose** | 342 | 148 | 56,63% |
| **Fructose** | 183 | 64 | 64,89% |

### 3.3. Extract concentration

In order to increase cynaropicrin concentration in final extract, a post-concentration step was applied till a VRF of 2. Since, no increase on sugars rejection was verified during diananofiltration, on concentration step it was expected that not only cynaropicrin concentration would increase, but also sugars concentration, but at a lower rate comparatively to cynaropicrin. And a massic balance to the entire system, diananofiltration and post-concentration, shows that at the end of the diananofiltration experiment, there was a decrease of approximately 57% on glucose content, with an increase on glucose loss of 36.21%, achieving a final removal of glucose of 92.84%.

**Table 5. Global experimental massic balance for cynaropicrin, glucose and fructose**

| | **Weight (mg)** | | |
|---|---|---|---|
| | **Initial** | **Final** | **% weight loss** |
| **Cynaropicrin** | 2194 | 1890 | 13.84% |
| **Glucose** | 342 | 24 | 92.84% |
| **Fructose** | 183 | 8 | 95.50% |

### 3.4. Integrated process of extraction and fractionation

To the best of our knowledge, up until the moment, there is no other membrane able to separate compound with such close molecular weight dissolved in an organic solvent, such as ethanol. Considering a future integration of the total process of cynaropicrin recovery from *Cynara cardunculus* leaves by pulsed ultrasound assisted extraction, with removal of sugars and low molecular weight compounds, an integrated process in suggested, with originated permeate is then evaporated, for a total recovery of ethanol for prior use in extraction as well as wash solvent.

### 4. Conclusions

*Cynara cardunculus* leaves are a great source of cynaropicrin, with amounts that can achieve 455 mg/g extract [16]. Increase of biological and economical potential of cardoon leaves extracts can be obtained by extract fractionation and removal of compounds present in high amounts and with low biological potential, such as carbohydrates.

The method disclosed herein stands as an integrated process for carbohydrates removal from cardoon leaves ethanolic pulsed ultrasound assisted extraction, with a cynaropicrin minimum loss of 10% weight and prior extract concentration, trough solvent resistant nanofiltration.

According to molecular weight of cynaropicrin and carbohydrates present in cardoon extracts and considering that ethanol was used as extraction solvent, three membranes were selected, NF 90, NF 270 and Duramem 200. With Duramem 200 being selected due to its highest cynaropicrin rejection (98%) and highest predicted glucose and fructose depletion in diananofiltration (75 and 78% respectively).

Massic balance to experimental diananofiltration, shows that for a 5.73 diavolumes, a removal of 56.63% (glucose) and 64.89% (fructose) was achieved with 9.27% cynaropicrin loss. Post-concentration allowed globally an almost complete removal of glucose (92.84%) and fructose (95.50%) with a maximum cynaropicrin weight loss of 13.84%.

An integrated process with two final main streams, cynaropicrin enriched extract and sugars enriched extract, with ethanol recovery by evaporation is also envisioned, in order to reuse of ethanol applied in the diananofiltration for pulsed ultrasound assisted extraction, minimizing the environmental impact of fractionation process.

Several features are described hereafter that can each be used independently of one another or with any combination of the other features. However, any individual feature might not address any of the problems discussed above or might only address one of the problems discussed above. Some of the problems discussed above might not be fully addressed by any of the features described herein. Although headings are provided, information related to a particular heading, but not found in the section having that heading, may also be found elsewhere in the specification.

### Brief description of drawings

For easier understanding of this application, figures are attached in the annex that represent the preferred forms of implementation which nevertheless are not intended to limit the technique disclosed herein.

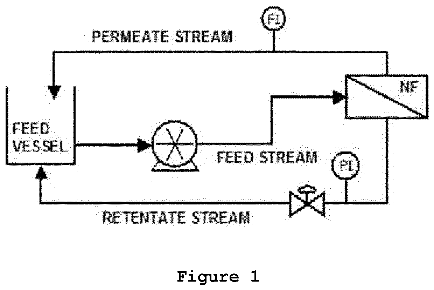

**Figure 1** illustrates the experimental nanofiltration setup (NF). PI and FI are pressure and flow-rate indicators, respectively.
**Figure 2** shows the experimental variation of permeate flux, Jᵥ (m³.m⁻².h⁻¹) with diavolumes for Duramem 200 membrane, during diananofiltration experiment.
**Figure 3** shows the experimental rejection (%) for cynaropicrin, glucose and fructose with diavolumes, during diananofiltration experiment.

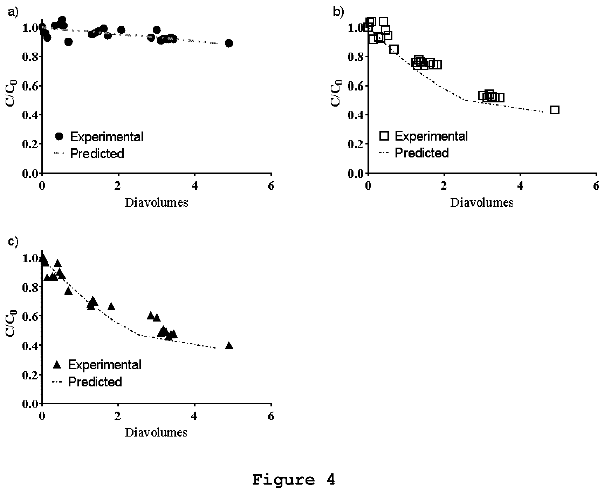

**Figure 4** shows the experimental and predicted normalized compound concentration with diavolumes. a) cynaropicrin; b) glucose; c) fructose.

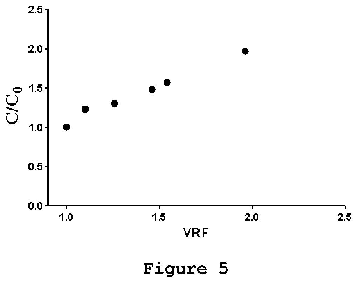

**Figure 5** shows the normalized cynaropicrin concentration with volume reduction factor (VRF).

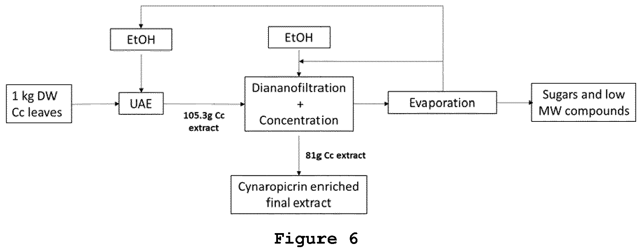

**Figure 6** illustrates the integrated process for cynaropicrin enriched PUAE *Cynara cardunculus* extract. In the illustrated example, 1 kg of dried cardoon leaves are extracted with ethanol as extraction solvent, with a cardoon extract production of 105.3 g. Extract produced is purified by membrane separation processes using diananofiltration and concentration steps, with ethanol as buffer solution. From purification step, two streams are obtained, one cynaropicrin enriched final extract stream with 81 g cardoon extract and another stream with sugars and low molecular weight compounds. The last one is obtained after solvent evaporation, where ethanol recovered can be applied as diananofitlration buffer as well as extraction solvent.

### Best mode for carrying out the invention

Now, preferred embodiments of the present application will be described in detail.

The preferred embodiment of the present patent application concerns a method for obtaining a cynaropicrin-rich extract comprising the following steps:
- Grounding of air dried *Cynara cadunculus;*
- Pulsed Ultrasonic assisted extraction (PUAE);
- Diananofiltration of the extract obtained in the previous step at a transmembrane pressure between 12 and 40 bar and a crossflow velocity between 0.5 and 1.5 m/s, wherein the membranes used for the filtration have a molecular weight cut-off between 100 and 400 Da;
wherein the retentate is an extract rich in cynaropicrin.

In one embodiment, the method for obtaining a cynaropicrin-rich extract described above further comprises a post-concentration step; wherein the permeate is removed until volume reduction factor (VRF) of 2 is achieved.

In one embodiment, the Ultrasonic assisted extraction step is performed with a duty cycle of 25%, a Solid/Liquid ratio of 1/27, extraction temperature of 44 °C and amplitude of 67% during 30 minutes.

In one embodiment, the membrane used in the diananofiltration step is made of modified polyimide or polyamide thin-film composite.

In one embodiment, the diananofiltration step is performed at a temperature between 25 and 40 °C.

This description is of course not in any way restricted to the forms of implementation presented herein and any person with an average knowledge of the area can provide many possibilities for modification thereof without departing from the general idea as defined by the claims. The preferred forms of implementation described above can obviously be combined with each other. The following claims further define the preferred forms of implementation.

## Claims

1. Method for obtaining a cynaropicrin-rich extract comprising the following steps:
- Grounding of air dried *Cynara cadunculus;*
- Pulsed Ultrasonic assisted extraction (PUAE);
- Diananofiltration of the extract obtained in the previous step at a transmembrane pressure between 12 and 40 bar and a crossflow velocity between 0.5 and 1.5 m/s, wherein the membranes used for the filtration have a molecular weight cut-off between 100 and 400 Da; wherein the retentate is an extract rich in cynaropicrin.

2. Method for obtaining a cynaropicrin-rich extract according to claim 1, wherein the ultrasonic assisted extraction step is performed with a duty cycle of 25%, a Solid/Liquid ratio of 1/27, extraction temperature of 44 °C and amplitude of 67% during 30 minutes.

3. Method for obtaining a cynaropicrin-rich extract according to any of claims 1-2, wherein the membrane used in the diananofiltration step is made of modified polyimide or polyamide thin-film composite.

4. Method for obtaining a cynaropicrin-rich extract according to any of claims 1-3, wherein the diananofiltration step is performed at a temperature between 25 and 40 °C.
